(19) Europäisches Patentamt
European Patent Office
Office européen des brevets

(11) **EP 4 524 559 A1**

(12) **EUROPEAN PATENT APPLICATION**
published in accordance with Art. 153(4) EPC

(43) Date of publication:
**19.03.2025   Bulletin 2025/12**

(21) Application number: **23803224.7**

(22) Date of filing: **07.03.2023**

(51) International Patent Classification (IPC):
*G01N 27/27* (2006.01)   *G01N 27/416* (2006.01)

(52) Cooperative Patent Classification (CPC):
**G01N 27/27; G01N 27/416**

(86) International application number:
**PCT/JP2023/008584**

(87) International publication number:
**WO 2023/218738 (16.11.2023 Gazette 2023/46)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **09.05.2022   JP 2022077029**

(71) Applicant: **HITACHI HIGH-TECH CORPORATION
Tokyo 105-6409 (JP)**

(72) Inventors:
• **MATSUSHITA, Yufuku**
**Tokyo 100-8280 (JP)**
• **KISHIOKA, Atsushi**
**Tokyo 100-8280 (JP)**
• **MIYAKE, Masafumi**
**Tokyo 105-6409 (JP)**

(74) Representative: **Mewburn Ellis LLP
Aurora Building
Counterslip
Bristol BS1 6BX (GB)**

(54)   **ANALYZING DEVICE, AND STATE DETECTING METHOD**

(57)   Provided is a technique for specifying an abnormal cause without requiring information of a known potential waveform in an analyzing device using an ion-selective electrode.

An analyzing device configured to analyze characteristics of a liquid having conductivity includes: a flow path through which liquid is supplied, an electrode provided in the flow path and in contact with the liquid, a liquid feeding mechanism configured to introduce the liquid into the flow path, an electric signal acquiring unit configured to acquire an electric signal output from the electrode, a storage unit configured to store a model in which the liquid in the flow path, the electrode, and the liquid feeding mechanism are expressed as an electric circuit, and a circuit simulator unit configured to simulate the electric signal on the basis of the model. The analyzing device estimates an electrical state change of the liquid in the flow path, the electrode, and the liquid feeding mechanism by simulating the electric signal in the circuit simulator unit.

*FIG. 4*

EP 4 524 559 A1

**Description**

Technical Field

The present invention relates to an analyzing device and a state detecting method.

Background Art

In order to quickly and easily measure the concentration of ions (electrolytes) such as potassium, sodium, and chloride in a biological sample liquid including blood, a plurality of ion-selective electrodes (ISE) corresponding to ions to be detected are mounted on an analyzing device.

The electrolyte analyzing unit having the ion-selective electrode is mounted on, for example, an automatic analyzing device. The automatic analyzing device is suitably used alone or as an element of a biochemical automatic analyzing device or the like in order to perform clinical examinations automatically, quickly, and continuously.

The ion-selective electrode is used in combination with a reference electrode. The activity (concentration) of a target ion is obtained by measuring a potential difference generated between the ion-selective electrode and the reference electrode. In the field of clinical examination, it is highly necessary to quantify the concentration of an electrolyte contained in blood that is a biological sample liquid, particularly a specimen such as serum, plasma, or urine. In some cases, these specimens are directly measured using an ion-selective electrode, that is, measured using a so-called undiluted method. A so-called diluted method may be used in which a predetermined amount of diluent is added to a predetermined amount of specimen, mixed, and diluted, and then measured using an ion-selective electrode.

The diluted method has features that the required amount of a specimen is small, the concentration of coexisting substances such as proteins and lipids in the measurement liquid is low, the influence of contamination by the coexisting substances is small, and the stability of the ion-selective electrode is high. Therefore, in analysis of electrolyte concentration using an automatic analyzing device, a combination of a flow-cell type ion-selective electrode and a diluted method is currently the mainstream. A container called a dilution tank is used to dilute the specimen. The diluted specimen (measurement liquid) prepared in the dilution tank is sent to the flow-cell type ion-selective electrode through a pipe and measured. The internal standard solution is dispensed into the dilution tank alternately with the specimen, and alternately measured with the specimen.

The electrolyte concentration in a living body is usually maintained in a narrow concentration range, and even a slight change in concentration is significant clinically or therapeutically. Therefore, an ion-selective electrode is required to have extremely high measurement accuracy, and various techniques have been developed to reduce measurement errors as much as possible.

For example, Patent Literature 1 describes a method of specifying a defective component by referring to a potential at a drive timing of a component as a method of specifying a cause of a measurement error of an ion-selective electrode.

Citation List

Patent Literature

PTL 1: JP 2021-018141 A

Summary of Invention

Technical Problem

There are various causes of the error of the measured value, such as bubble mixing into a flow path between the ion-selective electrode and the reference electrode, vibration of the liquid in the flow path, and mixing of electrical noise into the measurement system. A normal potential is not output due to an abnormality of the device that causes these.

In the conventional analyzing device described in Patent Literature 1, an abnormal cause of the analyzing device is specified based on a change in measurement potential. In this method, since the abnormal cause is determined based on the characteristic of the change in the measured potential (potential waveform), the relationship between the characteristic of the potential waveform and the abnormal cause needs to be clarified in advance.

Therefore, the present disclosure provides a technique for specifying an abnormal cause without requiring information of a known potential waveform in an analyzing device using an ion-selective electrode.

Solution to Problem

An example of an analyzing device according to the present invention is
an analyzing device configured to analyze characteristics of a liquid having conductivity, the analyzing device including:

a flow path through which liquid is supplied;
an electrode provided in the flow path and in contact with a liquid;
a liquid feeding mechanism configured to introduce liquid into the flow path;
an electric signal acquiring unit configured to acquire an electric signal output from the electrode;
a storage unit configured to store a model in which the liquid in the flow path, the electrode, and the liquid feeding mechanism are expressed as an electric circuit; and
a circuit simulator unit configured to simulate the electric signal on the basis of the model, in which
the analyzing device is configured to estimate an electrical state change of the liquid in the flow path, the electrode, and the liquid feeding mechanism by simulating the electric signal in the circuit simulator unit.

An example of a state detecting method according to the present invention is

a state detecting method executed by an analyzing device configured to analyze characteristics of a liquid having conductivity,
the analyzing device including:

a flow path through which liquid is supplied;
an electrode provided in the flow path and in contact with a liquid;
a liquid feeding mechanism configured to introduce liquid into the flow path;
an electric signal acquiring unit configured to acquire an electric signal output from the electrode;
a storage unit configured to store a model in which the liquid in the flow path, the electrode, and the liquid feeding mechanism are expressed as an electric circuit; and
a circuit simulator unit configured to simulate the electric signal on a basis of the model, and
the state detecting method including the steps of:

acquiring, by the electric signal acquiring unit, a potential waveform on a basis of a potential related to liquid supplied to the flow path;
acquiring, by the circuit simulator unit, an approximate waveform on the basis of the model and the acquired potential waveform, and acquiring a parameter related to a voltage, an electric resistance, a capacitance, and an inductance related to the approximate waveform;
comparing, by the analyzing device, the acquired parameter with threshold information related to the parameter stored in the storage unit; and
estimating, by the analyzing device, an electrical state change of the analyzing device on the basis of a result of the comparing step.

This specification contains the disclosure of Japanese Patent Application No. 2022-077029 on which priority of the present application is based.

Advantageous Effects of Invention

The analyzing device according to the present disclosure can analyze an abnormal cause in the analyzing device using an ion-selective electrode.
The problems, configurations, and effects other than those described above will be clarified from the description of the embodiments below.

Brief Description of Drawings

FIG. 1 is a schematic view illustrating an analyzing device according to a first embodiment.
FIG. 2 is an equivalent circuit of the analyzing device according to the first embodiment.
FIG. 3 is a simulation result of a potential waveform according to the first embodiment.
FIG. 4 is a flowchart of an analyzing method according to the first embodiment.

FIG. 5 is a flowchart of an analyzing method according to a second embodiment.
FIG. 6 is a schematic view illustrating an analyzing device according to a third embodiment.
FIG. 7 is a schematic view illustrating an analyzing device according to a fourth embodiment.
FIG. 8 is a schematic view illustrating an analyzing device according to a fifth embodiment.

Description of Embodiments

Hereinafter, embodiments of the present invention will be described with reference to the accompanying drawings.

[First embodiment]

< Configuration Example of Analyzing Device >

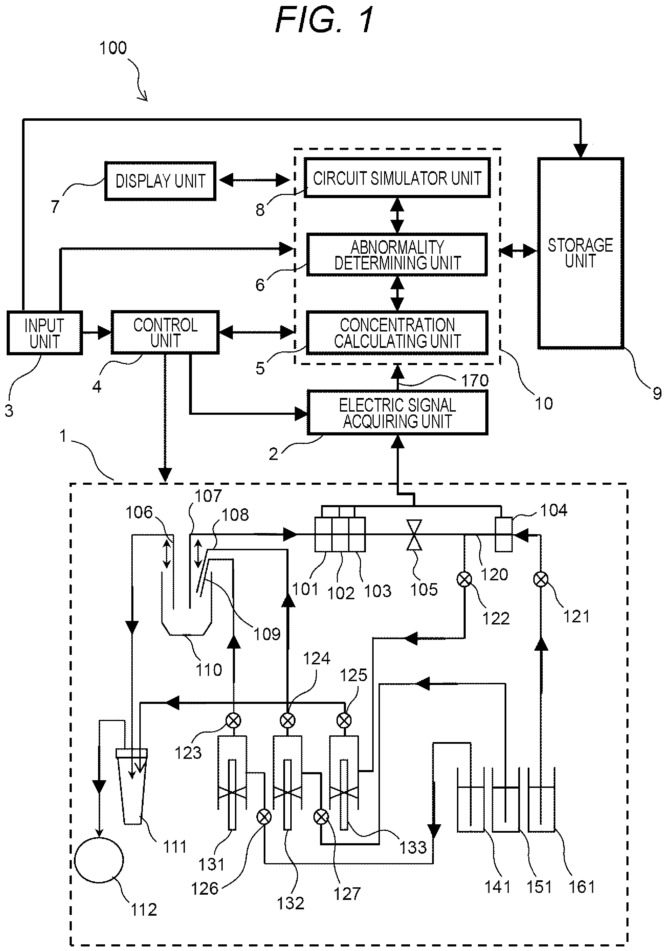

FIG. 1 is a schematic diagram illustrating an analyzing device 100 according to a first embodiment. The analyzing device 100 is an analyzing device that analyzes characteristics of a liquid having conductivity.

As illustrated in FIG. 1, the analyzing device 100 includes an electrolyte analyzing unit 1, an electric signal acquiring unit 2, an input unit 3, a control unit 4, a concentration calculating unit 5, an abnormality determining unit 6, a display unit 7, a circuit simulator unit 8, and a storage unit 9.

The electrolyte analyzing unit 1 includes three types of ion-selective electrodes 101 to 103, a comparison electrode 104 (reference electrode), a pinch valve 105, a vacuum suction nozzle 106, a sipper nozzle 107, a diluent supply nozzle 108, an internal standard solution supplying nozzle 109, a dilution tank 110, a waste liquid tank 111, a vacuum pump 112, electromagnetic valves 121 to 127, an internal standard solution syringe pump 131, a diluent syringe pump 132, a sipper syringe pump 133, an internal standard solution bottle 141, a diluent bottle 151, and a comparison electrode solution bottle 161. For example, the ion-selective electrode 101 is a chlorine ion electrode, the ion-selective electrode 102 is a potassium ion electrode, and the ion-selective electrode 103 is a sodium ion electrode.

The analyzing device 100 includes a flow path through which liquid is supplied. For example, the above-described components in the electrolyte analyzing unit 1 are interconnected by a flow path.

The vacuum pump 112, the internal standard solution syringe pump 131, the diluent syringe pump 132, and the sipper syringe pump 133 are examples of a liquid feeding mechanism for introducing a liquid into the flow path. The liquid feeding mechanism may include other components (for example, a pinch valve 105, a vacuum suction nozzle 106, a sipper nozzle 107, a diluent supply nozzle 108, an internal standard solution supplying nozzle 109, a dilution tank 110, a waste liquid tank 111, a liquid communication part 120, electromagnetic valves 121 to 127, an internal standard solution bottle 141, a diluent bottle 151, a comparison electrode solution bottle 161, and the like) of the electrolyte analyzing unit 1.

The ion-selective electrodes 101 to 103 and the comparison electrode 104 are examples of electrodes provided in the flow path and in contact with the liquid. Among them, the ion-selective electrodes 101 to 103 are electrodes that respond to the concentrations of specific ions, respectively, and this makes it possible to measure specific ions. As the ion-selective electrodes 101 to 103, for example, a flow-cell type ion-selective electrode can be used. The number of the ion-selective electrodes 101 to 103 can be changed according to the number of ion species to be measured. The ion-selective electrodes 101 to 103 can be adapted to all ion species. The ion-selective electrodes 101 to 103 generate an electromotive force (potential) according to the ion concentration in the sample liquid.

A comparison electrode solution (reference solution) is contained in the comparison electrode solution bottle 161. The reference solution is introduced into the flow path of the comparison electrode 104 by the sipper syringe pump 133. As the reference solution, for example, a potassium chloride aqueous solution or the like can be used. The comparison electrode 104 generates a potential corresponding to the ion concentration in the comparison electrode solution.

An internal standard solution (IS) is contained in the internal standard solution bottle 141. The internal standard solution is dispensed into the dilution tank 110 by the internal standard solution syringe pump 131 and the internal standard solution supplying nozzle 109.

The specimen is dispensed into the dilution tank 110 by a sampling mechanism (not illustrated). A diluent is contained in the diluent bottle 151. The diluent is dispensed into the dilution tank 110 by the diluent syringe pump 132 and the diluent supply nozzle 108 and mixed with the specimen.

As described above, the internal standard solution or the mixed liquid of the specimen and the diluent is introduced into the dilution tank 110 as the sample liquid to be analyzed.

Here, an operation of filling the sample liquid filled in the dilution tank 110 into the measurement flow path will be described. First, when the solution filled in the dilution tank 110 is introduced into the flow path of the ion-selective electrodes 101 to 103, the electromagnetic valve 121 and the electromagnetic valve 125 are closed, the pinch valve 105 and the electromagnetic valve 122 are opened, and the sipper nozzle 107 is lowered into the dilution tank 110 and sucked by the sipper syringe pump 133.

Subsequently, when the comparison electrode solution is introduced into the flow path of the comparison electrode 104, the electromagnetic valve 121 is opened and the pinch valve 105 is closed. Then, the comparison electrode solution is introduced from the comparison electrode solution bottle 161 into the flow path of the comparison electrode 104 by being sucked by the sipper syringe pump 133. In order to discharge the solution accumulated in the sipper syringe pump 133, the electromagnetic valve 122 is closed, the electromagnetic valve 125 is opened, and the liquid is pressure-fed to the sipper syringe pump 133.

The comparison electrode solution introduced into the flow path of the comparison electrode 104 and the sample liquid introduced into the ion-selective electrodes 101 to 103 are in contact with each other at the liquid communication part 120. The ion-selective electrodes 101 to 103 and the comparison electrode 104 are electrically connected to each other through the liquid.

After the sample liquid is introduced into the flow path of the ion-selective electrodes 101 to 103 and the comparison electrode solution is introduced into the flow path of the comparison electrode 104, the vacuum suction nozzle 106 is lowered and the vacuum pump 112 is driven. Thereby, the sample liquid (specimen or internal standard solution) remaining in the dilution tank 110 is sucked and discarded in the waste liquid tank 111. The comparison electrode solution introduced into the comparison electrode 104 is discarded in the waste liquid tank 111 by operating the electromagnetic valve 122, the vacuum pump 112, and the sipper syringe pump 133.

The potential difference between the comparison electrode 104 and each of the ion-selective electrodes 101 to 103 varies depending on the ion concentration of the analysis target in the sample liquid introduced into the flow path of the ion-selective electrodes 101 to 103. Hereinafter, the potential difference may be referred to as a potential or an electromotive force.

The electric signal acquiring unit 2 acquires electric signals output from the ion-selective electrodes 101 to 103 and the comparison electrode 104. The electric signal acquiring unit 2 outputs a potential measured based on the acquired electric signals to the concentration calculating unit 5, the abnormality determining unit 6, and the circuit simulator unit 8. The timing control function of the potential to be output and the functions of the concentration calculating unit 5, the abnormality determining unit 6, and the circuit simulator unit 8 are collectively referred to as a potential analyzing unit 10 in some cases.

The concentration calculating unit 5 calculates the electrolyte concentration in the sample liquid on the basis of the measurement result of the potential by the electric signal acquiring unit 2. As a method for measuring the electrolyte concentration, a known method can be adopted. For example, the abnormality determining unit 6 calculates a standard deviation, a difference between the maximum value and the minimum value, or an average value as an index of the soundness of the measured potential in a predetermined time range. The abnormality determining unit 6 compares the standard deviation, the difference between the maximum value and the minimum value, or the average value with the threshold information stored in the storage unit 9, thereby determining whether or not there is an abnormality.

The circuit simulator unit 8 simulates a potential change of the potential measured by the electric signal acquiring unit 2 and analyzes an abnormal cause of the analyzing device 100. Details of the simulation will be described later.

The input unit 3 is an input device such as a mouse, a keyboard, or a touch panel, for example. The input unit 3 is used by a user to input various data and instructions to the control unit 4, the concentration calculating unit 5, the abnormality determining unit 6, and the circuit simulator unit 8.

The control unit 4 controls operation of components in the analyzing device 100, potential measurement in the electric signal acquiring unit 2, and processing in the concentration calculating unit 5, the abnormality determining unit 6, and the circuit simulator unit 8. The control unit 4 receives an input from the input unit 3. Note that the processing in the control unit 4, the electric signal acquiring unit 2, the concentration calculating unit 5, the abnormality determining unit 6, and the circuit simulator unit 8 may be executed by one processor mounted on the analyzing device 100.

The display unit 7 displays results of processing in the concentration calculating unit 5, the abnormality determining unit 6, and the circuit simulator unit 8, a GUI screen, and the like.

The storage unit 9 stores data necessary for processing by the concentration calculating unit 5, the abnormality determining unit 6, and the circuit simulator unit 8, processing results, and the like. As data necessary for processing by the circuit simulator unit 8, the storage unit 9 stores data indicating a relationship between the potential measured by the electric signal acquiring unit 2 and the cause of the abnormality of the analyzing device 100.

FIG. 1 illustrates a form in which the storage unit 9 is incorporated in the analyzing device 100. The present invention is not limited thereto. The storage unit 9 can be used in any form such as a form in which the storage unit 9 is connected on the Internet. The storage unit 9 can also be used in a form in which the storage unit 9 can be detached from the analyzing device 100. The storage unit 9 can also be used in a form in which the storage medium is connected to the analyzing device 100. The storage unit 9 can also be used in a form in which the respective forms are combined.

The storage unit 9 and each of the concentration calculating unit 5, the abnormality determining unit 6, and the circuit simulator unit 8 are configured to be able to transmit and receive data, and for example, can be connected to a network or the Internet to acquire measurement data online.

For example, the electric signal acquiring unit 2, the circuit simulator unit 8, and the storage unit 9 may be connected via a communication network. As a more specific example, a connection 170 between the electric signal acquiring unit 2 and the circuit simulator unit 8 may be the Internet. In this way, functions can be distributed to achieve a more convenient configuration.

Note that all of the electric signal acquiring unit 2, the input unit 3, the control unit 4, the concentration calculating unit 5, the abnormality determining unit 6, the display unit 7, the circuit simulator unit 8, and the storage unit 9 are not necessarily incorporated in the analyzing device 100. Some of them may be provided in another device, and the other device and the analyzing device 100 may communicate with each other to exchange data.

< Error cause specification method >

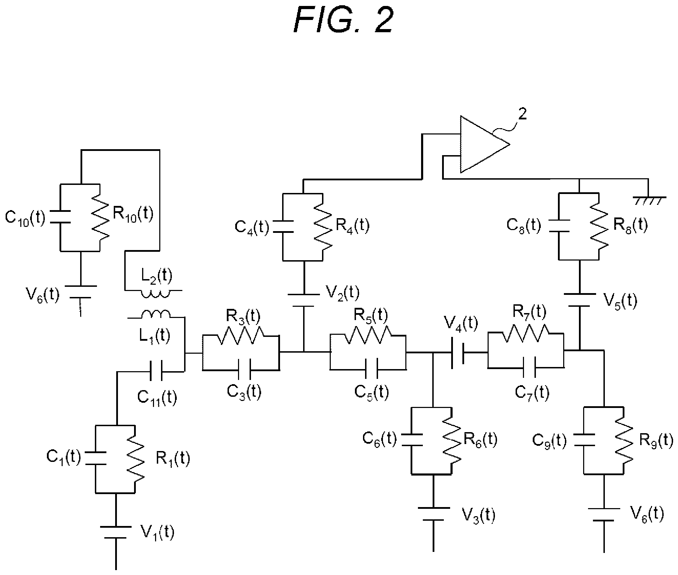

FIG. 2 is an equivalent circuit representing an electrical state of the ion-selective electrodes 101 to 103, the comparison electrode 104, the sipper nozzle 107, and a flow path through which the sample liquid, the reference liquid, and the waste liquid pass in the analyzing device 100. That is, FIG. 2 is an example of an equivalent circuit model representing the liquid, the electrode, and the liquid feeding mechanism in the flow path as an electrical circuit. As indices (parameters) of the electrical state, $C_x$ represents capacitance, $R_x$ represents electrical resistance, $V_x$ represents voltage, and $L_x$ represents inductance (x is an integer of 1 or more illustrated in FIG. 2). Note that a parameter $Z_x$ representing impedance may be used instead of or in addition to the parameters $C_x$, $R_x$, and $L_x$.

This equivalent circuit model is stored in, for example, the storage unit 9. The circuit simulator unit 8 can simulate the electric signal output from the ion-selective electrode 101 to 103 on the basis of such an equivalent circuit model. Note that the electric signal represents, for example, a potential between the ion-selective electrode 101 to 103 and the comparison electrode 104. This potential can be said to be a potential of the ion-selective electrode 101 to 103 expressed with the comparison electrode 104 as a reference.

Each parameter of the equivalent circuit model varies based on an electrochemical change accompanying driving of a component such as a measurement operation sequence, movement of the sample liquid in the flow path, and the like. Each variation can be defined as a function with the time t as a variable. The simulated potential $V_{sim}(t)$ expected to be acquired by the electric signal acquiring unit 2 is expressed by the following Formula 1 using functions of various parameters.

$$V_{sim}(t) = f(C_x(t), R_x(t), V_x(t), L_x(t), ...) \quad \text{(Formula 1)}$$

For example, in the equivalent circuit model of FIG. 2, $R_5(t)$, $R_6(t)$, $R_7(t)$, and $R_8(t)$ represent electric resistance in the flow path. When a bubble is mixed in the flow path, it can be considered that these values increase as compared with the normal values.

For example, $V_2(t)$ represents an electromotive force generated between the electrode and the sample liquid. $V_2(t)$ varies depending on not only the electrolyte concentration of the sample liquid but also the temperature.

For example, $V_4(t)$ represents a liquid-liquid interface potential generated at an interface where the sample liquid and the reference liquid come into contact with each other. $V_4(t)$ varies depending on the formation state of the liquid-liquid interface.

As described above, the change in the electrical state due to the flow path airtightness abnormality, the temperature adjustment abnormality, the liquid feeding abnormality, and the like is reflected in the potential output from the device. These changes are reflected to the potential in a form including the operation sequence of the components. Therefore, as the potential to be output, an equivalent circuit model in which dynamic behavior (time change behavior) of various components based on the operation sequence is expressed as an electric circuit in addition to a change as an electric circuit in an abnormal state can be used.

That is, the equivalent circuit model can express the dynamic behavior of the liquid, the electrode, and the liquid feeding mechanism in the flow path based on the operation sequence. In this case, the storage unit 9 may store a time chart of this operation sequence. In this way, simulation based on a specific operation sequence can be performed.

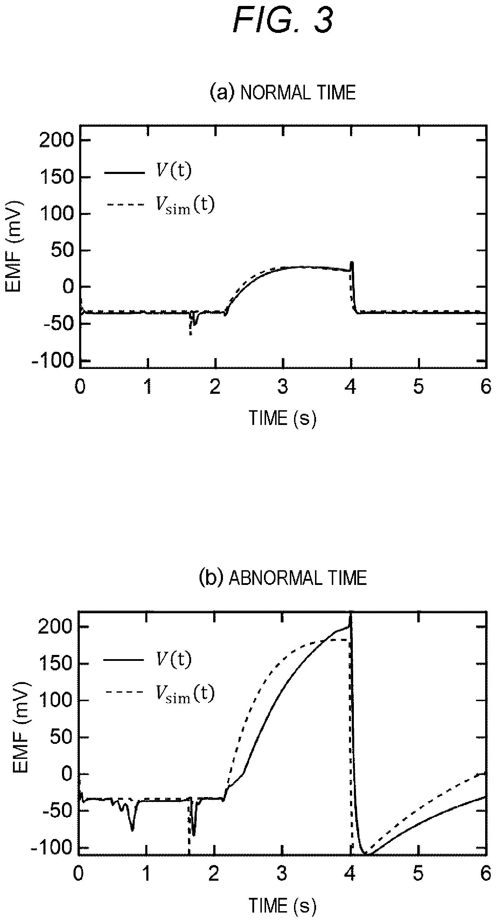

FIG. 3 illustrates a potential V(t) and a simulated potential $V_{sim}(t)$ approximated to the potential V(t) in the normal state (a) and the abnormal state (b). The simulated potential $V_{sim}(t)$ can be obtained, for example, by inputting specific values to $C_x(t)$, $R_x(t)$, $V_x(t)$, and $L_x(t)$ in a specific circuit as illustrated in FIG. 2.

As illustrated in FIG. 3(a), at the simulated potential $V_{sim}(t)$ that approximates V(t) at the normal time, it is considered that the values of all the parameters are within the normal range. As illustrated in FIG. 3(b), at the simulated potential $V_{sim}(t)$ that approximates V(t) at the abnormal time, it is considered that the value of any of the parameters is outside the normal range.

From the result of FIG. 3, since the simulated potential $V_{sim}(t)$ well matches the shape of the potential V(t), it can be seen that the electrical state of the analyzing device 100 can be substantially reflected. In this case, the electrical state of the device can be grasped by referring to $C_x(t)$, $R_x(t)$, $V_x(t)$, and $L_x(t)$ used to output $V_{sim}(t)$. In this manner, the analyzing device 100 can estimate an electrical state change of the liquid in the flow path, the electrode, the liquid feeding mechanism, the entire analyzing device 100, and the like by simulating the electric signal in the circuit simulator unit 8.

As a result, it is possible to estimate an abnormal cause or the like from the information regarding the electrical state. The analyzing device 100 estimates the electrical state change on the basis of the voltage, the electrical resistance, the capacitance, the inductance, and the impedance calculated by simulating the electric signal and the threshold information stored in the storage unit 9. Furthermore, it is possible to specify the abnormal cause on the basis of the estimated electrical state change. As a specific example, in a case where any of $C_x(t)$, $R_x(t)$, $V_x(t)$, and $L_x(t)$ is out of the normal range, it is possible to specify an abnormal portion on the basis of which parameter is out of the normal range, and specify an abnormal content on the basis of how the parameter is out of the normal range.

The equivalent circuit illustrated here represents a part of the analyzing device 100. An equivalent circuit model including other components can be created as necessary. In addition, a change in a circuit configuration due to an electrochemical phenomenon occurring at a solid-liquid interface or a liquid-liquid interface can also be introduced into this equivalent circuit model. For example, in any of $C_x(t)$, $R_x(t)$, $V_x(t)$, and $L_x(t)$ illustrated in FIG. 2, a temporal change due to such an electrochemical phenomenon may be defined, and/or new $C_x(t)$, $R_x(t)$, $V_x(t)$, and $L_x(t)$ representing a temporal change due to such an electrochemical phenomenon may be added. In this manner, the equivalent circuit model may represent a change in circuit configuration due to an electrochemical phenomenon occurring at a solid-liquid interface or a liquid-liquid interface. In this way, estimation in consideration of various electrochemical phenomena can be performed.

When a phenomenon model that cannot be directly expressed as a parameter is introduced in the equivalent circuit, the parameter may be expressed by using an index related to $C_x(t)$, $R_x(t)$, $V_x(t)$, and $L_x(t)$ The equivalent circuit model can be changed on the basis of the operation sequence, and there are a method of setting and changing a model created in advance on the basis of time chart information of the operation sequence stored in the storage unit 9, and a method of changing by manual input from the input unit 3. For example, the analyzing device 100 may have a circuit simulator input unit that receives an input of a parameter or an equivalent circuit model used for simulation with respect to the circuit simulator unit 8 or the storage unit 9. The input unit 3 can function as such a circuit simulator input unit. In this way, more various models can be supported.

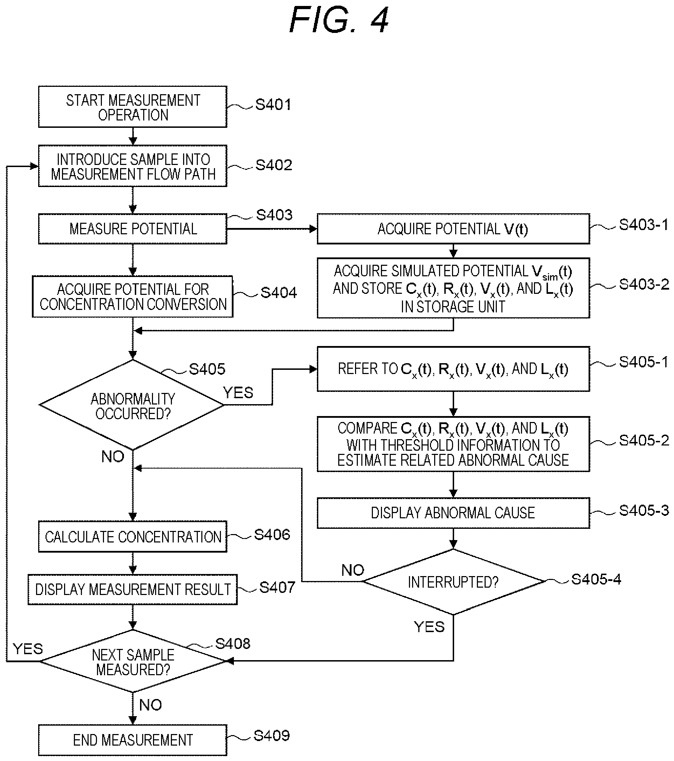

FIG. 4 is a flowchart illustrating a method of specifying a specific abnormal cause using the circuit simulator unit 8 having the function of the circuit simulation. This flowchart illustrates an example of a state detecting method executed by the analyzing device 100.

First, in step S401, when the user inputs an operation start instruction from the input unit 3, the control unit 4 drives the electrolyte analyzing unit 1 to start the measurement operation.

In step S402, the electrolyte analyzing unit 1 introduces the sample liquid dispensed into the dilution tank 110 into the flow path including the ion-selective electrodes 101 to 103.

In step S403, the electric signal acquiring unit 2 measures a potential difference between each of the ion-selective electrodes 101 to 103 and the comparison electrode 104. The electric signal acquiring unit 2 outputs the potential difference to the concentration calculating unit 5, the abnormality determining unit 6, and the circuit simulator unit 8. Here, the electric signal acquiring unit 2 acquires a potential waveform on the basis of a potential related to the liquid supplied to the flow path. At this time, the potential to be output may target all the timings at which the components are operating, or may be limited to a certain time region.

The potential output to the circuit simulator unit 8 is treated as the potential V(t) for abnormality analysis in step S403-1. A simulated potential $V_{sim}(t)$ approximated to the potential waveform is acquired and stored in the storage unit in step S403-2. For example, a set of $C_x(t)$, $R_x(t)$, $V_x(t)$, and $L_x(t)$ that gives $V_{sim}(t)$ closest to V(t) is acquired. In this way, the circuit simulator unit 8 acquires the approximate waveform on the basis of the equivalent circuit model and the acquired potential waveform. The circuit simulator unit 8 acquires parameters regarding the voltage, the electrical resistance, the capacitance, the inductance, and the impedance related to the approximate waveform. As an approximation method, a least squares method, a maximum likelihood estimation method, a Bayesian estimation method, or the like is used. This can be set in advance by the input unit 3 and/or the control unit 4.

In step S404, the concentration calculating unit 5 acquires a potential for concentration conversion. In step S405, the abnormality determining unit 6 determines the presence or absence of abnormality by comparing the standard deviation, the difference between the maximum value and the minimum value, the average value, and the like as indices of the soundness of the potential with respective pieces of threshold information stored in the storage unit. Note that, in the abnormality determination here, the values of $C_x(t)$, $R_x(t)$, $V_x(t)$, and $L_x(t)$ acquired in step S403-2 are unnecessary (these values can be used as a matter of course) .

In a case where it is determined in step S405 that there is no abnormality, the process proceeds to step S406. In step S406, the concentration calculating unit 5 calculates the concentration. In step S407, the concentration calculated is displayed on the display unit 7.

In a case where it is determined in step S405 that there is an abnormality, $C_x(t)$, $R_x(t)$, $V_x(t)$, and $L_x(t)$ stored in the storage unit in step S403-2 are referred to and acquired in step S405-1.

Next, the analyzing device 100 compares each parameter acquired in step S405-1 with threshold information regarding each parameter stored in the storage unit in step S405-2. Then, the analyzing device 100 estimates an electrical state change of the liquid in the flow path, the electrode, the liquid feeding mechanism, the entire analyzing device 100, and the like on the basis of the comparison result. For example, the abnormal cause can be specified on the basis of the presence or absence of a parameter exceeding the threshold, the type of the parameter exceeding the threshold, the degree of exceeding the threshold, and the like.

In particular, in the present embodiment, the analyzing device 100 estimates the electrical state change of the liquid in the flow path, the electrode, and the liquid feeding mechanism on the basis of the comparison between the electric signal output from the ion-selective electrode 101 to 103 and the comparison electrode 104 and the approximate waveform. That is, estimating the electrical state change of the analyzing device 100 includes estimating the electrical state change of the liquid in the flow path, the electrode, and the liquid feeding mechanism. This makes it possible to perform estimation based on a specific temporal change in potential.

A relationship between each parameter and a related component is stored in the storage unit. When a certain parameter exceeds a threshold, an abnormal state (abnormal cause) of the related component and the component considered to cause the threshold to be exceeded can be specified.

A plurality of thresholds may be defined for the same parameter, and in that case, different abnormal causes can be specified according to each threshold excess. Since there may be a plurality of abnormal causes, a plurality of abnormal causes (or the possibility thereof) may be specified for one piece of threshold information. Information such as an occurrence frequency can be stored together for each abnormal cause.

A relationship between parameters is also stored in the storage unit 9. An abnormal cause can be specified on the basis of a combination of parameters exceeding a threshold. The above-described relationship can be arbitrarily set and changed.

As a method of specifying the abnormal cause of the component, in addition to this method, a plurality of equivalent circuit models expressing an abnormal state in which the values of various parameters are set to values outside the normal range are stored in advance in the storage unit. The abnormal cause can be specified on the basis of the evaluation of the approximation between the abnormal simulated potential $V_{sim\text{-}fault}(t)$ and the potential $v(t)$ simulated on the basis of each model. The selection of the abnormality determining method can be set in advance by the input unit 3 and/or the control unit 4.

In step S405-3, the specified abnormal cause is displayed on the display unit 7. At this time, not only the abnormal cause but also the type, the value, the threshold, the potential $V(t)$, and the simulated potential $V_{sim}(t)$ of the abnormal parameter can be set to be displayed on the display unit.

In step S405-4, it is selected whether or not to interrupt the measurement. In a case of no interruption, the process proceeds to step S406, and in a case of interruption, the process proceeds to step S408. In step S408, it is selected whether or not to measure the next sample liquid, and in a case of measurement, the process proceeds to step S402, and the measurement is restarted. In a case of no measurement, the measurement ends in step S409.

< Technical effects >

As described above, the analyzing device 100 according to the present embodiment obtains the simulated potential $V_{sim}(t)$ approximate to the potential $V(t)$ and compares each parameter of $V_{sim}(t)$ with the threshold information. The analyzing device 100 can specify the cause of the measurement error on the basis of the relationship between the plurality of parameters and the excess degree with respect to the threshold. As a result, it is possible to inform the user of a portion that may cause a measurement error and an improvement method. Therefore, it is possible to reduce the time required to specify the portion that causes the error and to reduce the work load on the user.

In this method, the potential $V(t)$ is analyzed based on the circuit theory. Accordingly, interpretability and interpretability for the specification result of the abnormal cause are high as compared with the conventional method of analyzing on the basis of an empirical potential change.

In the conventional method of performing analysis on the basis of an empirical potential change, when a change occurs in the device configuration, it is necessary to newly collect abnormal potential data and reconstruct an analysis algorithm associated with abnormal cause specification. Since the present method conforms to the circuit theory, even when the device configuration is changed, the change of the algorithm is completed only by changing the equivalent circuit for the changed portion and adjusting the threshold of each parameter. Thus, the robustness against the device configuration change is high.

The analyzing device 100 of the present embodiment can specify the cause of the abnormality in parallel with the measurement and the calculation of the ion concentration. Therefore, the measurement throughput is not reduced.

[Second embodiment]

In the first embodiment, the method of specifying the "cause" of the abnormality from the simulated potential $V_{sim}$ (t)) obtained from the potential V(t) and the parameter has been described. On the other hand, in the second embodiment, a method of detecting an abnormality "sign" from the simulated potential $V_{sim}$(t) obtained from the potential V(t) and its parameter will be described.

< Analyzing device >

As the analyzing device of the second embodiment, the same one as the analyzing device 100 described in the first embodiment can be used.

< Analyzing method >

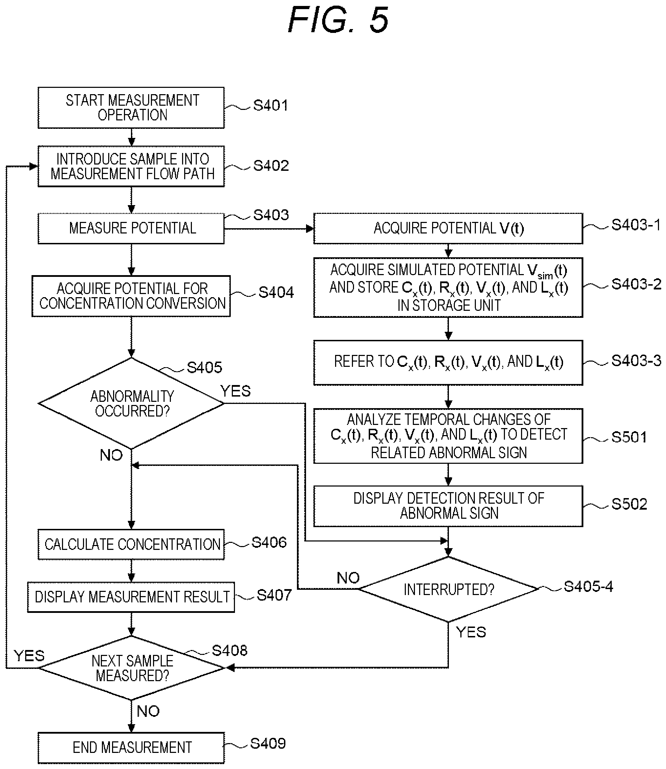

FIG. 5 is a flowchart illustrating an analyzing method according to the second embodiment. In FIG. 5, steps similar to those illustrated in FIGS. 3 and 4 (first embodiment) are denoted by the same reference numerals. Hereinafter, differences from the flowchart of FIG. 4 will be described in detail.
First, in parallel with the operations in and after step S404 executed after step S403, steps S403-1, S402-1, and S403-3 are continuously executed by the circuit simulator unit 8. Step S403-3 can be similar to, for example, step S405-1 of FIG. 4. Thereafter, in step S501, temporal changes of $C_x(t)$, $R_x(t)$, $V_x(t)$, and $L_x(t)$ are analyzed to detect an abnormal sign.
At this time, in the analysis of the temporal change, a change point is detected and comparison with threshold information for sign detection is performed. A specific operation can be performed similarly to, for example, S405-2 in FIG. 4 (the first embodiment). That is, the storage unit stores a relationship between each parameter and a related component, and when a certain parameter exceeds a threshold, the related component and an abnormal state of the component considered to cause the threshold exceeding can be specified. When an abnormal state is identified, it can be said that a sign that the abnormality will occur in the future has been detected. In this manner, the analyzing device 100 detects an abnormal sign on the basis of the estimated electrical state change. Thereafter, in step S502, the detection result of the abnormal sign is displayed on the display unit 7.

< Technical effects >

As described above, the analyzing device of the present embodiment can detect an abnormal sign of the device by obtaining the simulated potential $V_{sim}$(t) approximated to the potential V(t) and analyzing the temporal change of each parameter of $V_{sim}$(t). As a result, it is possible to notify the user of a cause portion or disturbance that may cause an error cause in the future before an abnormality occurs.
Since the maintenance time can be planned in advance, it is possible to avoid sudden stop of the analyzing device 100 and to perform the maintenance management work at a time when the operation rate of the analyzing device 100 is low. Therefore, it is possible to contribute to improvement of the measurement throughput.

[Third embodiment]

In the first embodiment, the method of specifying the abnormal cause from the parameters obtained by the simulation of the potential obtained from the ion-selective electrode 101 to 103 has been described. On the other hand, in the third embodiment, it will be described a method of detecting an abnormal cause or an abnormal sign by simulating potentials obtained at a plurality of electrodes provided in a plurality of flow paths such as a flow path through which a sample liquid or a reference liquid passes.

< Analyzing device >

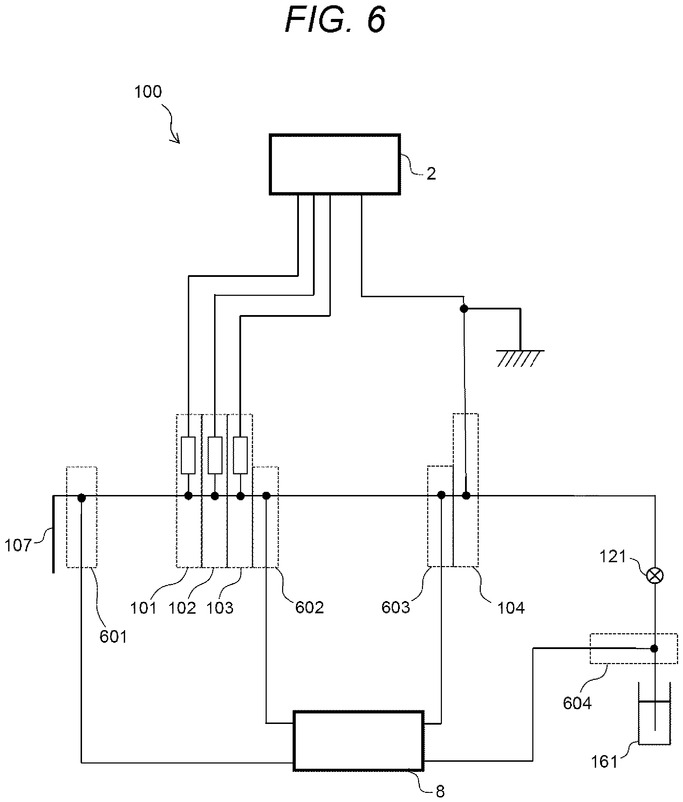

FIG. 6 illustrates a configuration in the analyzing device 100 according to the third embodiment. In addition to the ion-selective electrodes 101 to 103 and the comparison electrode 104, electrodes 601, 602, 603, and 604 are provided in a flow path through which the sample liquid and the reference liquid flow. Each output is connected to the circuit simulator unit 8. Each potential may be obtained based on a ground potential (GND) in the device.
In the first embodiment, the ion-selective electrodes 101 to 103 are provided at the same site or adjacent sites in the flow path. In the present embodiment, any or all of the electrodes 601, 602, 603, and 604 are provided at a site different from the ion-selective electrodes 101 to 103 in the flow path or at a position other than the flow path.

< Analyzing method >

The analyzing device of the third embodiment uses the analyzing methods described in the first and second embodiments. The analyzing device of the third embodiment performs the specification of the cause of potential abnormality and the detection of the abnormal sign using the parameters obtained by the simulation of the potential of electrodes including the electrodes 601, 602, 603, and 604. That is, the analyzing device 100 includes a plurality of electrodes, and the circuit simulator unit 8 can simulate an electric signal for each of the plurality of electrodes.

The number of equivalent circuit models may be one or more. For example, the storage unit 9 may store a plurality of equivalent circuit models, and each equivalent circuit model may correspond to a different electrode. In that case, the circuit simulator unit 8 may acquire an approximate waveform for each of the equivalent circuit models. The analyzing device 100 may estimate the electrical state change by evaluating the approximation between each approximate waveform and the electric signal of the corresponding electrode.

< Technical effects >

It is possible to specify an abnormal cause or detect an abnormal sign by simulating potentials obtained from a plurality of electrodes provided in a plurality of flow paths such as a flow path through which a sample liquid or a reference liquid passes. As a result, the accuracy of abnormality determination by simulation is improved as compared with the simulation of the potential of the electrode at one place, and more accurate specification of the abnormal cause and detection of the abnormal sign can be realized.

When a plurality of equivalent circuit models are used, a more precise model specialized for each electrode can be used.

[Fourth embodiment]

In the fourth embodiment, an ammeter that measures a current flowing through a flow path is provided. A method will be described in which an abnormal cause is specified and an abnormal sign is detected by simulating the measured current.

< Analyzing device >

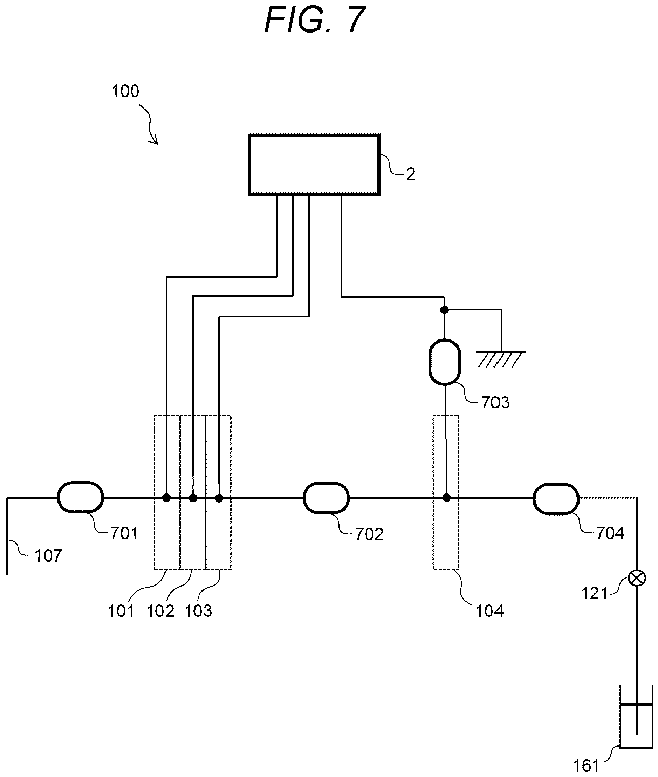

FIG. 7 illustrates a configuration of an analyzing device 100 according to the fourth embodiment. Ammeters 701, 702, 703, and 704 are provided. The ammeters 701, 702, 703, and 704 measure a current flowing through a flow path through which a sample liquid or a reference liquid flows or through a conductive wire. As the ammeter, a movable coil type, a rectification type, a current force type, an induction type, an electrostatic type, a thermoelectric type, a movable iron piece type, or the like is used. These ammeters are examples of means for measuring the current flowing through the flow path, the electrode, or the liquid feeding mechanism.

< Analyzing method >

The analyzing device of the fourth embodiment acquires a parameter by simulating the current of each ammeter in addition to the analyzing methods described in the first and second embodiments. That is, the circuit simulator unit 8 can simulate the current on the basis of the equivalent circuit model. The equivalent circuit model may be a model in which a parameter $I_x$ representing a current is further added to the model illustrated in FIG. 2.

The analyzing device 100 estimates an electrical state change by simulating an electric signal (for example, measured by the ion-selective electrodes 101 to 103) and a current (for example, measured by any or all of the ammeters 701, 702, 703, and 704) in the circuit simulator unit 8. Then, the analyzing device 100 specifies a potential abnormal cause and detects an abnormal sign based on the estimation result.

< Technical effects >

As described above, the analyzing device of the present embodiment can specify the abnormal cause or detect the abnormal sign by simulating the current provided in the flow path through which the sample liquid or the reference flow passes in addition to the simulation of the potential. As a result, information that cannot be analyzed only by the potential information can be compensated by the current information, so that the accuracy of abnormality determination by simulation is improved. Thus, more accurate specification of an abnormal cause and detection of an abnormal sign can be realized.

[Fifth embodiment]

In the fifth embodiment, it will be described a method of specifying an abnormal cause or detecting an abnormal sign by measuring a disturbance such as temperature, vibration, or static electricity in a flow path through which a sample liquid or a reference liquid flows or in the vicinity thereof, and simulating a potential including the influence of the measured disturbance.

< Analyzing device >

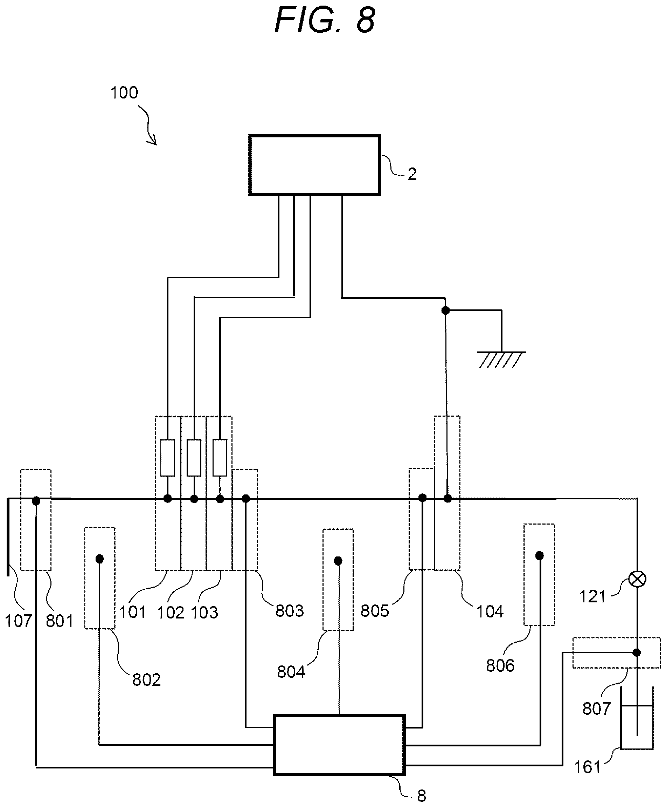

FIG. 8 illustrates a configuration of an analyzing device 100 according to the fifth embodiment. Sensors 801, 802, 803, 804, and 805 are provided. The sensors 801, 802, 803, 804, and 805 measure a disturbance in a flow path through which a sample liquid or a reference liquid flows or in the vicinity thereof.

For example, the analyzing device 100 includes means for measuring a temperature (for example, ambient temperature) related to the flow path, the electrode, or the liquid feeding mechanism. The circuit simulator unit 8 can simulate an electric signal on the basis of an equivalent circuit model and the temperature.

Examples of the disturbance measured by these sensors include vibration (acceleration and/or angular velocity), a potential related to static electricity, and the like in addition to temperature. Parameters representing these disturbances can be incorporated into an equivalent circuit using, for example, $C_x(t)$, $R_x(t)$, $V_x(t)$, $L_x(t)$, and the like in FIG. 2. A specific method for converting the disturbance into these electrical parameters can be appropriately designed by a person skilled in the art. In the analyzing device 100, a position to which the sensors 801, 802, 803, 804, and 805 should be attached in order to measure these disturbances can also be appropriately designed by those skilled in the art.

< Analyzing method >

The analyzing device of the fifth embodiment performs the specification of the potential abnormal cause and the detection of the abnormal sign using the parameters obtained by simulating the potential including the influence of the disturbance by the sensor in addition to the analyzing methods described in the first and second embodiments.

< Technical effects >

As described above, the analyzing device according to the present embodiment measures the disturbance such as the temperature, vibration, and static electricity, in the flow path through which the sample liquid or the reference flow passes or in the vicinity thereof. The analyzing device according to the present embodiment simulates the potential including the influence of the measured disturbance. Thus, the analyzing device according to the present embodiment can specify the abnormal cause or detect the abnormal sign. As a result, the disturbance information that cannot be analyzed only by the potential information can be compensated by the sensor information. Thereby, the accuracy of abnormality determination by simulation is improved, and it is possible to realize more accurate specification of an abnormal cause and detection of an abnormal sign.

[Sixth embodiment]

In the sixth embodiment, it will be described a method of generating a pseudo potential waveform reflecting various states of a device in a case where it is difficult to collect field data.

< Analyzing device >

As the analyzing device of the sixth embodiment, the same one as the analyzing device 100 described in the first to fifth embodiments can be used. That is, the sixth embodiment can be implemented in combination with any of the first to fifth embodiments.

< Analyzing method >

First, the simulated potential $V_{sim}(t)$ corresponding to the normal time is obtained by the method described in Example 1. Thereafter, any of the parameters of the simulated potential $V_{sim}(t)$ is changed to a value outside the normal range, and a new simulated potential $V_{sim}(t)$ is obtained using the changed parameter. This new simulated potential $V_{sim}(t)$ can be treated as an example of an abnormal potential waveform. In this manner, the analyzing device 100 generates a simulation signal that simulates a specific abnormal state.

As a result, a plurality of examples of abnormal potential waveforms can be collected. It is also possible to randomly input a value within a range having a predetermined width (however, outside the normal range) as the value of the parameter to create a data set of an abnormal potential waveform.

A plurality of simulated potentials $V_{sim}(t)$ when the values of the parameters are randomly changed within the normal range may be acquired, whereby a data set of a normal potential waveform can also be created.

< Technical effects >

As described above, the analyzing device of the present embodiment can generate an example of a normal or abnormal potential waveform. The generated potential waveform can be treated as an alternative to the field data. As a result, even when it is difficult to collect the field data, it is possible to consider an abnormality analyzing method from the waveform information. Specifically, it is possible to study a classification model of a potential waveform by supervised learning, specify an abnormal cause, and study threshold setting used for abnormal sign detection.

Reference Signs List

1 electrolyte analyzing unit
2 electric signal acquiring unit
3 input unit
4 control unit
5 concentration calculating unit
6 abnormality determining unit
7 display unit
8 circuit simulator unit
9 storage unit
10 potential analyzing unit
100 analyzing device
101 to 103 ion-selective electrode (electrode)
104 comparison electrode
105 pinch valve (liquid feeding mechanism)
106 vacuum suction nozzle (liquid feeding mechanism)
107 sipper nozzle (liquid feeding mechanism)
108 diluent supply nozzle (liquid feeding mechanism)
109 internal standard solution supplying nozzle (liquid feeding mechanism)
110 dilution tank (liquid feeding mechanism)
111 waste liquid tank (liquid feeding mechanism)
112 vacuum pump (liquid feeding mechanism)
120 liquid communication part (liquid feeding mechanism)
121 to 127 electromagnetic valve (liquid feeding mechanism)
131 internal standard solution syringe pump (liquid feeding mechanism)
132 diluent syringe pump (liquid feeding mechanism)
133 sipper syringe pump (liquid feeding mechanism)
141 internal standard solution bottle (liquid feeding mechanism)
151 diluent bottle (liquid feeding mechanism)
161 comparison electrode solution bottle (liquid feeding mechanism)
170 connection
601 to 604 electrode
701 to 704 ammeter
801 to 805 sensor

All publications, patents, and patent applications cited herein are hereby incorporated by reference in their entirety.

**Claims**

1. An analyzing device configured to analyze characteristics of a liquid having conductivity, the analyzing device comprising:

a flow path through which liquid is supplied;
an electrode provided in the flow path and in contact with a liquid;
a liquid feeding mechanism configured to introduce liquid into the flow path;
an electric signal acquiring unit configured to acquire an electric signal output from the electrode;
a storage unit configured to store a model in which the liquid in the flow path, the electrode, and the liquid feeding mechanism are expressed as an electric circuit; and
a circuit simulator unit configured to simulate the electric signal on a basis of the model, wherein
the analyzing device is configured to estimate an electrical state change of the liquid in the flow path, the electrode, and the liquid feeding mechanism by simulating the electric signal in the circuit simulator unit.

2. The analyzing device according to claim 1, wherein

the model expresses dynamic behavior of the liquid in the flow path, the electrode, and the liquid feeding mechanism based on an operation sequence, and
the storage unit is configured to store a time chart of the operation sequence.

3. The analyzing device according to claim **1,** wherein the electrode includes an ion-selective electrode configured to respond to a concentration of a specific ion.

4. The analyzing device according to claim 1, wherein the model expresses a change in a circuit configuration due to an electrochemical phenomenon occurring at a solid-liquid interface or a liquid-liquid interface.

5. The analyzing device according to claim 1, wherein the analyzing device is configured to specify an abnormal cause based on the estimated state change.

6. The analyzing device according to claim 1, wherein the analyzing device is configured to detect an abnormal sign based on the estimated state change.

7. The analyzing device according to claim 1, wherein the analyzing device is configured to estimate the state change on a basis of a voltage, an electric resistance, a capacitance, and an inductance calculated by the simulation of the electric signal, and threshold information stored in the storage unit.

8. The analyzing device according to claim 1, further comprising a circuit simulator input unit configured to receive an input of a parameter or the model used for simulation with respect to the circuit simulator unit or the storage unit.

9. The analyzing device according to claim **1,** wherein the electric signal acquiring unit, the circuit simulator unit, and the storage unit are connected via a communication network.

10. The analyzing device according to claim **1,** wherein

the analyzing device includes a plurality of the electrodes, and
the circuit simulator unit is configured to simulate the electric signal for each of the electrodes.

11. The analyzing device according to claim 1, further comprising a unit configured to measure a current flowing through the flow path, the electrode, or the liquid feeding mechanism, wherein

the circuit simulator unit is configured to simulate the current on a basis of the model, and
the analyzing device is configured to estimate the state change by simulating the electric signal and the current in the circuit simulator unit.

12. The analyzing device according to claim 1, further comprising a unit configured to measure a temperature related to the flow path, the electrode, or the liquid feeding mechanism, wherein
the circuit simulator unit is configured to simulate the electric signal on a basis of the model and the temperature.

13. The analyzing device according to claim **1,** wherein the analyzing device is configured to generate a simulation signal that simulates a specific abnormal state.

14. A state detecting method executed by an analyzing device configured to analyze characteristics of a liquid having conductivity,

the analyzing device including:

a flow path through which liquid is supplied;

an electrode provided in the flow path and in contact with a liquid;

a liquid feeding mechanism configured to introduce liquid into the flow path;

an electric signal acquiring unit configured to acquire an electric signal output from the electrode;

a storage unit configured to store a model in which the liquid in the flow path, the electrode, and the liquid feeding mechanism are expressed as an electric circuit; and

a circuit simulator unit configured to simulate the electric signal on a basis of the model, and

the state detecting method comprising the steps of:

acquiring, by the electric signal acquiring unit, a potential waveform on a basis of a potential related to liquid supplied to the flow path;

acquiring, by the circuit simulator unit, an approximate waveform on a basis of the model and the acquired potential waveform, and acquiring a parameter related to a voltage, an electric resistance, a capacitance, and an inductance related to the approximate waveform;

comparing, by the analyzing device, the acquired parameter with threshold information related to the parameter stored in the storage unit; and

estimating, by the analyzing device, an electrical state change of the analyzing device on a basis of a result of the comparing step.

15. The state detecting method according to claim 14, wherein

the model expresses dynamic behavior of the liquid in the flow path, the electrode, and the liquid feeding mechanism based on an operation sequence, and

the storage unit stores a time chart of the operation sequence.

16. The state detecting method according to claim 14, wherein the step of estimating an electrical state change of the analyzing device includes a step of estimating an electrical state change of a liquid in the flow path, the electrode, and the liquid feeding mechanism.

17. The state detecting method according to claim 14, wherein

the storage unit stores a plurality of the models,

the circuit simulator unit is configured to acquire the approximate waveform for each of the models, and

the analyzing device is configured to estimate the state change by evaluating approximation between each of the approximate waveforms and the electric signal.

18. The state detecting method according to claim 14, further comprising a step of specifying, by the analyzing device, an abnormality cause on a basis of the estimated state change.

19. The state detecting method according to claim 14, further comprising a step of detecting, by the analyzing device, an abnormal sign on a basis of the estimated state change.

# FIG. 1

# FIG. 2

# FIG. 3

### (a) NORMAL TIME

### (b) ABNORMAL TIME

EP 4 524 559 A1

# FIG. 4

START MEASUREMENT OPERATION —S401

INTRODUCE SAMPLE INTO MEASUREMENT FLOW PATH —S402

MEASURE POTENTIAL —S403

ACQUIRE POTENTIAL $V(t)$ —S403-1

ACQUIRE SIMULATED POTENTIAL $V_{sim}(t)$ AND STORE $C_x(t)$, $R_x(t)$, $V_x(t)$, AND $L_x(t)$ IN STORAGE UNIT —S403-2

ACQUIRE POTENTIAL FOR CONCENTRATION CONVERSION —S404

ABNORMALITY OCCURRED? —S405

YES

REFER TO $C_x(t)$, $R_x(t)$, $V_x(t)$, AND $L_x(t)$ —S405-1

COMPARE $C_x(t)$, $R_x(t)$, $V_x(t)$, AND $L_x(t)$ WITH THRESHOLD INFORMATION TO ESTIMATE RELATED ABNORMAL CAUSE —S405-2

DISPLAY ABNORMAL CAUSE —S405-3

NO

CALCULATE CONCENTRATION —S406

DISPLAY MEASUREMENT RESULT —S407

NO INTERRUPTED? —S405-4

YES

NEXT SAMPLE MEASURED? —S408

YES

NO

END MEASUREMENT —S409

18

# FIG. 5

START MEASUREMENT OPERATION —S401

INTRODUCE SAMPLE INTO MEASUREMENT FLOW PATH —S402

MEASURE POTENTIAL —S403 → ACQUIRE POTENTIAL V(t) —S403-1

ACQUIRE SIMULATED POTENTIAL $V_{sim}(t)$ AND STORE $C_x(t)$, $R_x(t)$, $V_x(t)$, AND $L_x(t)$ IN STORAGE UNIT —S403-2

ACQUIRE POTENTIAL FOR CONCENTRATION CONVERSION —S404

REFER TO $C_x(t)$, $R_x(t)$, $V_x(t)$, AND $L_x(t)$ —S403-3

S405 ABNORMALITY OCCURRED? — YES / NO

ANALYZE TEMPORAL CHANGES OF $C_x(t)$, $R_x(t)$, $V_x(t)$, AND $L_x(t)$ TO DETECT RELATED ABNORMAL SIGN —S501

DISPLAY DETECTION RESULT OF ABNORMAL SIGN —S502

CALCULATE CONCENTRATION —S406

DISPLAY MEASUREMENT RESULT —S407

NO — INTERRUPTED? —S405-4 / YES

S408 NEXT SAMPLE MEASURED? — YES / NO

END MEASUREMENT —S409

# FIG. 6

# FIG. 7

# FIG. 8

## INTERNATIONAL SEARCH REPORT

| International application No. |
| --- |
| **PCT/JP2023/008584** |

### A. CLASSIFICATION OF SUBJECT MATTER

***G01N 27/27***(2006.01)i; ***G01N 27/416***(2006.01)i

FI: G01N27/416 366D; G01N27/27 D; G01N27/416 351A; G01N27/416 351J

According to International Patent Classification (IPC) or to both national classification and IPC

### B. FIELDS SEARCHED

Minimum documentation searched (classification system followed by classification symbols)

G01N27/27-27/49

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | JP 2021-018141 A (HITACHI HIGH TECH CORP.) 15 February 2021 (2021-02-15) entire text, all drawings | 1-19 |
| A | JP 2014-086313 A (CALSONIC KANSEI CORP.) 12 May 2014 (2014-05-12) claims 1, 3 | 1-19 |
| A | JP 2020-041968 A (HITACHI HIGH-TECHNOLOGIES CORPORATION) 19 March 2020 (2020-03-19) entire text, all drawings | 1-19 |
| A | JP 2019-158448 A (PRIMEARTH EV ENERGY CO., LTD.) 19 September 2019 (2019-09-19) entire text, all drawings | 1-19 |
| A | JP 2010-501873 A (METTLER-TOLEDO AG) 21 January 2010 (2010-01-21) entire text, all drawings | 1-19 |

☐ Further documents are listed in the continuation of Box C.　　☑ See patent family annex.

| | |
| --- | --- |
| *　Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **24 April 2023** | **16 May 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2023/008584**

| Patent document cited in search report | | | Publication date (day/month/year) | Patent family member(s) | | | Publication date (day/month/year) |
|---|---|---|---|---|---|---|---|
| JP | 2021-018141 | A | 15 February 2021 | EP entire text, all drawings CN WO | 4001910 114127549 2021/014695 | A1 A A1 | |
| JP | 2014-086313 | A | 12 May 2014 | (Family: none) | | | |
| JP | 2020-041968 | A | 19 March 2020 | US entire text, all drawings WO EP CN | 2021/0318266 2020/054473 3851842 112654862 | A1 A1 A1 A | |
| JP | 2019-158448 | A | 19 September 2019 | (Family: none) | | | |
| JP | 2010-501873 | A | 21 January 2010 | US entire text, all drawings WO CN | 2009/0157338 2008/025776 101135662 | A1 A1 A | |

Form PCT/ISA/210 (patent family annex) (January 2015)

24

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021018141 A

- JP 2022077029 A